Europäisches Patentamt

European Patent Office

Office européen des brevets

(11) Veröffentlichungsnummer: **0 256 155**
**A1**

(12) **EUROPÄISCHE PATENTANMELDUNG**

(21) Anmeldenummer: **86111293.6**

(22) Anmeldetag: **14.08.86**

(51) Int. Cl.4: **A61B 5/04 , A61N 1/08**

(43) Veröffentlichungstag der Anmeldung:
**24.02.88 Patentblatt 88/08**

(84) Benannte Vertragsstaaten:
**DE FR GB IT NL SE**

(71) Anmelder: **Hellige GmbH**
**Postfach 728 Heinrich-von-Stephan-Strasse 4**
**D-7800 Freiburg im Breisgau(DE)**

(72) Erfinder: **Streu, Benno**
**Turnseestrasse 1**
**D-7800 Freiburg i. Br.(DE)**

(74) Vertreter: **Patentanwälte TER MEER - MÜLLER - STEINMEISTER**
**Mauerkircherstrasse 45**
**D-8000 München 80(DE)**

(54) Operationsmonitor mit Hochfrequenz-Warneinrichtung.

(57) Zur Überwachung von Körperfunktionen eines Patienten während eines operativen Eingriffs mittels HF-Chirurgie erfasst eine HF-Überwachungseinrichtung (5 bis 17) die über die Leitungen (13) von den Überwachungselektroden (4) in einem Operationsmonitor (1) zugeführten Hochfrequenzspannungen. Eine den HF-Spannungen proportionale Richtspannung wird in einem Komparator (10) gegen eine einstellbare Referenzspannung verglichen. Überschreitet die Richtspannung die Referenzspannung, so wird ein Warnsignal (9) erzeugt, das den operierenden Arzt auf mögliche HF-Verbrennungsgefahr hinweist, so daß Gegenmaßnahmen ergriffen werden können.

FIG.1

EP 0 256 155 A1

## Operationsmonitor mit Hochfrequenz-Warneinrichtung

Die Erfindung betrifft einen Operationsmonitor zur Überwachung von Körperfunktionen eines Patienten während einer Operation durch Abgreifen einer gewünschten Überwachungsgröße, wie Herzfrequenz, Puls, Körpertemperatur oder dergleichen, mittels einer am oder im Körper des Patienten anzulegenden Meßelektrode.

Die Anwendung der HF-Chirurgie ist nicht problemfrei. Die dem Patienten applizierte elektrische Energie wird hier nicht direkt als Stromdurchgang durch den Körper gefährlich, sondern indirekt als in Wärme umgeformte Energie. Der Patient wird Teil eines Hochfrequenz-Stromkreises. Wegen der fehlenden Reizwirkung hochfrequenter Ströme können bei diesem Verfahren außerordentlich hohe Ströme über den Körper geleitet werden; über den Körper geleitet werden; an Stellen mit kleinem Stromquerschnitt tritt eine hohe Stromdichte; Hitzeentwicklung, Platzen der Zellen und damit der erwünschte Schneideffekt auf. Bei der Anwendung dieses Verfahrens besteht für den Arzt jedoch die Aufgabe, alle ungewollten Stromübergangstellen zu vermeiden, da sie Verbrennungsgefahren für den Patienten bedeuten können.

Die Ursachen, die zu HF-Verbrennungen im Operationssaal führen, sind jedoch sehr komplex, und es verwundert daher nicht, daß trotz stetiger Unterrichtung des mit der Operationsvorbereitung betrauten Personals Verbrennungen der genannten Art hinsichtlich ihrer Häufigkeit bei weitem an der Spitze von Zwischenfällen in Operationssälen liegen.

Angestrebt ist, daß der Patient, der mittels einer großflächigen Neutral-oder Erdelektrode geerdet wird, elektrisch immer passiv bleibt, auch dann, wenn aus dem "HF-Messer" hohe Ströme in den Körper eintreten. Bedingt durch den Rückfluß der Ströme über den Patientenkörper, Haut/Elektroden-Übergang und Erdleitung mit wechselnden Leitwerten kann dieser Idealzustand natürlich nicht erreicht werden, selbst dann nicht, wenn den üblichen Empfehlungen entsprechend die großflächige Erdungselektrode in möglichst kurzem Abstand von mit dem HF-Messer zu bearbeitenden Operationsfeld angelegt wird. Das Phänomen des additiven Spannungsabfalls auf der Strecke über den Patientenkörper, über die Anschlußleitung bis hin zum HF-Generator, wird häufig von dem eine Operation vorbereitenden Krankenhauspersonal nicht total überblickt und daher auch nicht erkannt als Hauptursache aller HF-Zwischenfälle, daß nämlich der Patient als Ganzes während des HF-Schnitts gegenüber seiner neutralen Umgebung eine beträchtliche HF-Spannung annehmen kann. Über jeden direkten oder kapazitiven Erdkontakt fließen dann Ströme ab, und diese führen, wenn dabei unverträgliche Stromdichten an der Hautoberfläche auftreten, zu den bekannten Überhitzungen, insbesondere an den Aufliegestellen, z. B. am Rücken (Schulter und Gesäß).

Weiterhin besteht Verbrennungsgefahr durch HF-Ströme unter den zur Operationsüberwachung angelegten Überwachungselektroden und -abnehmern, wie beispielsweise EKG-Elektroden oder Temperaturabnehmern. Trotz der heute vorwiegend zum Einsatz kommenden sogenannten "Floating-Input"-Verstärker sind solche Elektroden oder Abnehmer hochfrequenzmäßig gut geerdete Teile. Gerade deshalb kann es aber nicht verwundern, daß es auch unter solchen Geräteteilen zu Verbrennungen kommt.

Da eine vollkommene Isolierung des Patienten gegen Erde nicht möglich ist, wenn er während der HF-Anwendung mit anderen geerdeten Geräten in Verbindung stehen muß, wurden immer wieder Richtlinien für das richtige Anlegen der Überwachungs-oder Monitorelektroden ausgesprochen. Auch werden seit einiger Zeit spezielle Patientenleitungen zur per-operativen Überwachung angeboten, bei denen zwischen der Elektrode und der Übertragungsleitung Widerstände oder Drosselspulen eingeschaltet sind. Mit solchen Leitungen kann der Strom, der über die Elektroden fließt, nachhaltig reduziert werden. Trotzdem kommt es in ungünstigen Fällen und unter Einhaltung aller Operationsbedingungen bei bestimmten Arten von Operationen immer wieder zu den gefürchteten HF-Verbrennungen.

Der Erfindung liegt damit die Aufgabe zugrunde, eine Einrichtung zu schaffen, die den operierenden Arzt bzw. das Operationsüberwachungspersonal warnt, wenn für den Patienten HF-Gefahren drohen.

Die im Patentanspruch 1 angegebene erfindungsgemäße Lösung der gestellten Aufgabe geht dahin, die HF-Spannungen, die über die Elektrodenleitungen in den Operationsmonitor eintreten, zu messen, mit einer Schwelle zu vergleichen und bei Überschreiten eines vorzugsweise voreinstellbaren Grenzwerts ein Warnsignal auszulösen, das eine "HF-Gefahr" signalisiert. Der Arzt kann dann bei Aufleuchten eines Warnlichts oder Ertönen eines Warnsignals sofort eine wirksame Gegenmaßnahme ergreifen, z. B. die am HF-Messer eingespeiste Energie zurücknehmen, den Abstand zwischen HF-Messer und Überwachungselektrode vergrößern oder die Lage und Feuchtigkeit der Neutralelektrode überprüfen lassen. Das Erlöschen der Warnung signalisiert dann den Erfolg der Gegenmaßnahmen.

Die Erfindung bedeutet eine wesentliche Verbesserung der Operationsüberwachung durch Operationsmonitoren.

Gemäß einer bevorzugten Ausführungsform ist auch eine Kombination der erfindungsgemäßen HF-Überwachungseinrichtung zur Gewinnung einer der HF-Spannung am Eingang des Operationsmonitors proportionalen Größe mit bekannten Entstörschaltungen in solchen Monitoren zur Ausfilterung von unerwünschten HF-Anteilen möglich.

Die Erfindung und vorteilhafte Einzelheiten werden nachfolgend unter Bezug auf die Zeichnungen anhand eines Ausführungsbeispiels näher erläutert. Es zeigen:

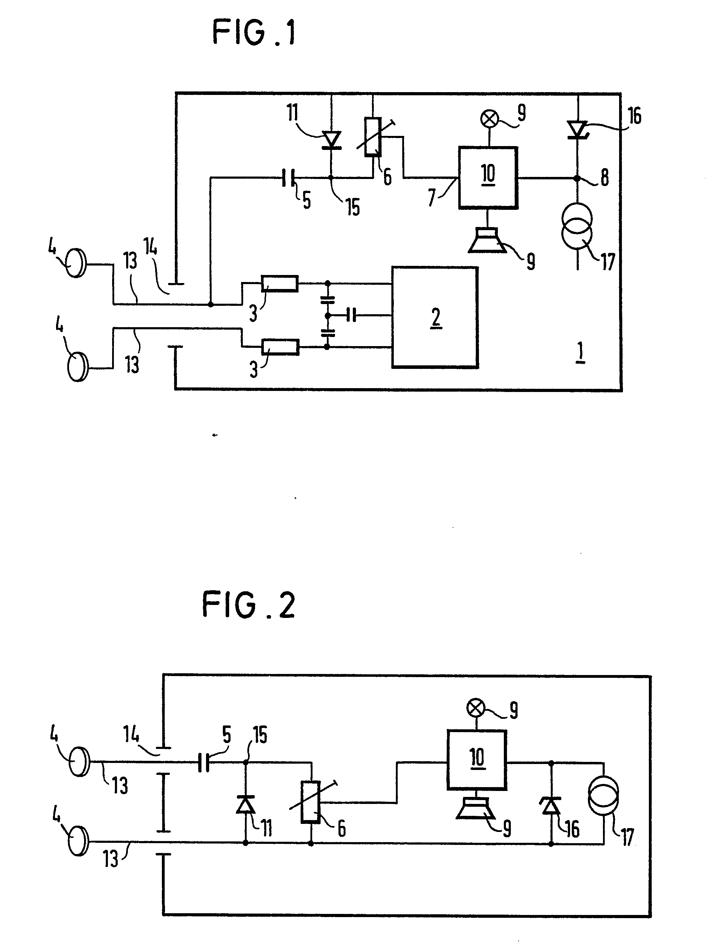

Figur 1 in schematischer Darstellung einen Operationsmonitor oder ein einschiebbares Modul zur Überwachung einer bestimmten Körperfunktion innerhalb eins Operationsmonitor mit einer Mehrzahl von einschiebbaren Modulen, und Figur 2 eine als selbständige Einheit ausgeführtes HF-Warngerät gemäß der Erfindung.

Das allgemein mit 1 angegebene Gerät enthält z. B. einen EKG-Verstärker 2, dem ein Filter 3 zur Abtrennung unerwünschter HF-Komponenten vorgeschaltet sein kann. An der Eintrittsstelle 14 der von EKG-Elektroden 4 kommenden Lei tung(en) kann induktiv oder, wie dargestellt, über einen Kondensator 5 und einen Gleichrichter 11 eine der Hochfrequenz proportionale Richtspannung am Punkt 15 gewonnen werden, von der zweckmäßigerweise eine geeignete Teilspannung, z. B. über einen ohmschen Widerstand, insbesondere ein Potentiometer 6, abgegriffen wird und dem Vergleichseingang 7 eines Komparators 10 zugeführt wird. Im Komparator 10 wird die gleichgerichtete HF-(Teil-)Spannung mit einer beispielsweise über eine Zener-Diode 16 stabilisierten, von einer Quelle 17 gelieferten Schwellenspannung am Punkt 8 verglichen. Übersteigt die Richtspannung die Schwellenspannung am Punkt 8, so wird ein Triggersignal für eine oder mehrere Warneinrichtungen 9 bereitgestellt, die, wie im dargestellten Beispiel gezeigt, akustische und/oder optische Warneinrichtungen sein können. Diese Warneinrichtungen 9 werden so lange aktiviert, als die Richtspannung die Schwellenspannung übersteigt.

Mit der Erfindung läßt sich die HF-Spannung des Patienten gegen die geerdete Umgebung zuverlässig überwachen, die bei überschreiten einer bestimmten Höhe zentrale Ursache von HF-Verbrennungen werden kann.

Die im erläuterten Ausführungsbeispiel als Zusatzeinrichung für einen Operationsmonitor beschriebene HF-Überwachungseinrichtung ist nach Figur 2 auch als selbständiges HF-Warngerät ausführbar, wobei dann eine Erd-(Referenz-) Elektrode und eine Meßelektrode vorzusehen sind.

## Ansprüche

1. Operationsmonitor zur Überwachung von Körperfunktionen eines Patienten während einer Operation durch Abgreifen von Überwachungsgrößen, wie Herzfrequenz, Puls, Körpertemperatur oder dergleichen, mittels einer oder mehrerer am oder im Körper des Patienten anzulegenden Meßelektrode(n) (4) oder Abnehmer, **gekennzeichnet durch**

-eine bei Anwendung von HF-Chirurgie wirksame Einrichtung (5, 11) zur Erfassung und Gewinnung einer Vergleichgröße der über die Zuleitung(en) in den Monitor (1) gelangenden Hochfrequenzspannungen,

-eine Vergleichseinrichtung (10), in der die gewonnene Vergleichsgröße mit einem Schwellenwert verglichen wird,

und durch

-eine durch die Vergleichseinrichtung bei Überschreiten der Schwelle durch das erfaßte HF-Signal triggerbare Warneinrichtung (9).

2. Operationsmonitor nach Anspruch 1, **dadurch gekennzeichnet,** daß die HF-Spannungen an der Eintrittsstelle der von der Meßelektrode (4) oder dem Abnehmer kommenden Leitung(en) (13) in den Monitor (1) induktiv oder kapazitiv erfaßt, gleichgerichtet und über einen Spannungsteiler (6) als dem erfaßten HF-Spannungswert proportionale Größe auf den Vergleichseingang (7) der Vergleichseinrichtung (10) geschaltet werden.

3. Operationsmonitor nach Anspruch 2, **dadurch gekennzeichnet,** daß der Spannungsteiler (6) einstellbar ist.

4. Operationsmonitor nach einem der vorstehenden Ansprüche, **dadurch gekennzeichnet,** daß die der Vergleichseinrichtung (10) zugeführte Schwellenwert einstellbar ist.

5. HF-Warngerät als selbständiges Gerät zur Erzeugung eines Warnsignals bei Anwendung der Hochfrequenzchirurgie, wenn unzulässig hohe HF-Spannungen am Patientenkörper gegen das Erdpotential auftreten, **gekennzeichnet durch**

-eine mit dem Erdpotential zu verbindende Referenzelektrode,

-eine an einer von der Operationsstelle entfernt am oder im Patientenkörper anzubringende Meßelektrode,

-eine Einrichtung (5, 11) zur Erfassung der über die Meßelektrode gegen die Referenzelektrode in das Warngerät gelangenden Hochfrequenzspannungen,

-eine Vergleichseinrichtung (10), in der die HF-Spannungen gegen einen Schwellenwert verglichen werden,

und durch

-eine durch die Vergleichseinrichtung bei Überschreiten der Schwelle durch das erfaßte HF-Signal triggerbare Warneinrichtung (9).

# FIG .1

# FIG .2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| Y | DE-B-1 235 503 (SIEMENS AG)<br>* Spalte 1, Zeilen 1-11; Spalte 2, Zeilen 30-52; Spalte 3, Zeilen 7-15,23-35,43-53; Abbildung 1 *<br><br>--- | 1,2,5 | A 61 B 5/04<br>A 61 N 1/08 |
| Y | LU-A- 70 789 (MATBURN (HOLDINGS) LTD.)<br>* Seite 2, Zeile 8 - Seite 3, Zeile 10; Abbildung 1 * | 1,2,5 | |
| A | | 4 | |
| | --- | | |
| A | US-A-4 313 079 (Y.S. LEE)<br>* Zusammenfassung; Spalte 1, Zeile 62 - Spalte 2, Zeile 28; Abbildung 1 *<br><br>----- | 3,5 | |

**RECHERCHIERTE SACHGEBIETE (Int. Cl.4)**

A 61 B
A 61 N

Der vorliegende Recherchenbericht wurde für alle Patentansprüche erstellt.

| Recherchenort | Abschlußdatum der Recherche | Prüfer |
|---|---|---|
| DEN HAAG | 19-03-1987 | FERRIGNO, A. |